# EUROPEAN PATENT SPECIFICATION

(11) **EP 3 445 382 B1**
(45) Date of publication and mention of the grant of the patent: **09.09.2020**
(21) Application number: 17706820.2
(22) Date of filing: 24.02.2017
(51) Int. Cl.: A61K 36/38, A61Q 19/00, A61K 8/97, A61P 17/10

(54) **HARUNGANA MADAGASCARIENSIS LEAF EXTRACT FOR USE IN THE TREATMENT AND/OR PREVENTION OF ACNE**
EXTRAKT AUS HARUNGANA-MADAGASCARIENSIS-BLÄTTERN ZUR VERWENDUNG BEI DER BEHANDLUNG UND/ODER PROPHYLAXE VON AKNE
EXTRAIT DE FEUILLES DEHARUNGANA MADAGASCARIENSIS DESTINÉ À ÊTRE UTILISÉ DANS LE TRAITEMENT ET/OU LA PRÉVENTION DE L'ACNÉ

(30) Priority: 21.04.2016 EP 16166506
(43) Date of publication of application: 27.02.2019
(73) Proprietor: Société d'Exploitation de Produits pour les Industries Chimiques SEPPIC, 75321 Paris cedex 07 (FR)
(72) Inventor: PETIT, Virginie, 64140 Lons (FR); MOUROT, Béatrice, 64160 Saint-Castin (FR); HUMBERT, Virginie, 74140 Sciez (FR)
(74) Representative: Laigneau, Amandine
(86) International application number: PCT/EP2017/054399
(87) International publication number: WO 2017/182182

(56) References cited:
- WO-A2-2014/009874
- FR-A1- 2 997 016
- MOULARI B ET AL: "Isolation and in vitro antibacterial activity of astilbin, the bioactive flavanone from the leaves of Harungana madagascariensis Lam. ex Poir. (Hypericaceae)", JOURNAL OF ETHNOPHARMACOLOGY, ELSEVIER IRELAND LTD, IE, vol. 106, no. 2, 30 June 2006 (2006-06-30) , pages 272-278, XP027939647, ISSN: 0378-8741 [retrieved on 2006-06-30]
- OKEKE MI; IROEGBU CU; EBO PU: "Antibacterial activity of Harungana madagascariensis leaf extracts", PHYTOTHER RES., vol. 16, no. 2, March 2002 (2002-03), pages 174-9, XP002698502, cited in the application

## Description

The present invention relates to *Harungana madagascariensis* leaf extract for use in the treatment and/or prevention of acne. The disclosure also relates to a method of preparing *Harungana madagascariensis* leaf extract.

The Harungana genus belongs to the Hypericaceae (classified also Clusiaceae or Gutifferae) family, native to Madagascar and imported in Africa for its medicinal properties. *Harungana madagascariensis Lam. ex Poir.* (Haronga) is the sole member of the genus.

Harungana is traditionally used as a colorant, an anti-inflammatory and an antidiabetic medicine in Cameroon. Its Bark and roots are used against scabies, vermifuge, intestinal problems (leaves). Fresh gum is used for the treatment of scurf and eczema, as an antiseptic, antioxidant.
*Harungana madagascariensis Lam. ex Poir.* is well known for its topical antibacterial properties used in the elaboration of skin hygiene products. Aqueous decoctions of *Harungana madagascariensis Lam. ex Poir.* leaves have been described for the treatment of leprosy and cutaneous mycoses. Effectively this plant has a significant history in folk medicine used to treat various diseases and is recognized as having topical antimicrobial properties used in the elaboration of skin hygiene products.
WO 2014/009874 A2 discloses the use of a *Harungana madagascariensis* extract for promoting collagen synthesis and to protect the skin proteins from glycation.
FR 2997016 A1 concerns the use of an extract of *Harungana madagascariensis* in a moisturizing cosmetic composition that is useful for treating dry and/or dehydrated skins.

Okoli AS et al, teach a rationalization for the traditional use of "Harungana madagascariensis*"* leaf extracts for the treatment of gastrointestinal disorders.is directed towards the antibacterial activity of *Harungana madagascariensis* leaf extracts. More specifically, aqueous extracts of the leaf of *Harungana madagascariensis* were analyzed phytochemically and evaluated for antimicrobial activity against strains of *Bacillus subtilis, Staphylococcus aureus, Escherichia coli, Salmonella typhi* and *Pseudomonas aeruginosa.* Glycosides, tannins, saponins, flavonoids and alkaloids were detected in the plant material. *B*. *subtilis, E. coli* and *S*. *typhi,* but not *Ps*. *aeruginosa,* showed susceptibility at MICs of 2.0 mg/ml and 15.6 mg/ml; and MBCs of 2.0mg/ml to 3.9 mg/ml and 15.6mg/ml to 31.3 mg/ml, respectively, for the cold and hot extracts. *Staphylococcus aureus* showed susceptibility only to the hot extract. Concentrations of 2.5mg/ml to 10.0 mg/ml of the cold extract killed over 7 log₍₁₀₎ of the test bacterial population within 30-60 min of exposure. The hot extract needed higher concentrations and longer treatment to achieve similar levels of bacterial cell killing. The results provide a rationalization for the traditional use of *Harungana madagascariensis* leaf extracts for the treatment of gastrointestinal disorders [Okeke MI, Iroegbu CU and Ebo PU in: "Antibacterial activity of Harungana madagascariensis leaf extracts", Phytother Res. 2002 Mar; 16(2):174-9].

Further background reference is given in the following publications:
- Agbor GA, Kuate D and Oben JE, Pak J Biol Sci. 2007 Feb 15;10(4):537-44;
- Kisangau DP, Hosea KM, Joseph CC and Lyaruu HV, Afr J Tradit Complement Altern Med. 2007 Jun 10;4(4):510-23;
- Biapa PC, Agbor GA, Oben JE and Ngogang JY, Afr J Tradit Complement Altern Med. 2007 Jun 10;4(4):495-500;
- Moulari B, Pellequer Y, Lboutounne H, Girard C, Chaumont JP, Millet J, Muyard F, J Ethnopharmacol. 2006 Jun 30;106(2):272-8;
- EP 0 985 416 A2 and
- EP 1 452 167 A1.

Acne vulgaris is a long-term skin disease that occurs when hair follicles become clogged with dead skin cells and oil from the skin. *Propionibacterium acnes* is a relatively slow-growing, typically aerotolerant anaerobic, Gram-positive bacterium linked to the skin condition of acne. This bacterium is largely commensal and part of the skin flora present on most healthy adult humans' skin. P. acnes bacteria live deep within follicles and pores, away from the surface of the skin. In these follicles, P. acnes bacteria use sebum, cellular debris and metabolic byproducts from the surrounding skin tissue as their primary sources of energy and nutrients. Elevated production of sebum by hyperactive sebaceous glands (sebaceous hyperplasia) or blockage of the follicle can cause P. acnes bacteria to grow and multiply.

Many different treatments exist for acne, including alpha hydroxy acid, anti-androgen medications, antibiotics, antiseborrheic medications, azelaic acid, benzoyl peroxide, hormonal treatments, keratolytic soaps, nicotinamide, retinoids, and salicylic acid. They are believed to work in at least four different ways, including the following: anti-inflammatory effects, hormonal manipulation, killing P. acnes, and normalizing skin cell shedding and sebum production in the pore to prevent blockage.

The present disclosure has the object of providing a plant-based cosmetic composition. Accordingly, the disclosure relates to a composition comprising from 0.005 weight% to 0.250 weight% of *Harungana madagascariensis* leaf extract, based on the weight of the dry extract and of the total weight of the formulation, and one or more topically acceptable excipients and/or solvents.

The present invention has also the object of providing a plant-based treatment and/or prevention of acne. Accordingly, the present invention is directed towards *Harungana madagascariensis* leaf extract, and relates to a composition comprising from 0.005 weight% to 0.250 weight% of *Harungana madagascariensis* leaf extract, based on the weight of the dry extract and of the total weight of the formulation, for its use to treat and/or to prevent acne, in a method of treatment of the human body by therapy.

*Harungana madagascariensis* leaf extract can be converted in a known manner into the usual formulations such as pharmaceutical or cosmetic compositions or compositions used as food supplement (i.e. according to the concept of "beauty from within") or medical device. These may be liquid or solid formulations e.g. without limitation normal and enteric coated tablets, capsules, pills, powders, granules, elixirs, tinctures, solution, suspensions, suppositories, syrups, solid and liquid aerosols, emulsions, pastes, creams, ointments, milks, gels, salves, serums, foams, shampoos, sticks or lotions.

Preference is given to a pharmaceutical or cosmetic composition in a form of an aqueous solution, a white or coloured cream, ointment, milk, gel, salve, serum, foam, shampoo, stick, cream, paste, or lotion.

In general, the leaf extract can be provided as a formulation, such as a topical formulation. Preferably, the formulation comprises a pharmaceutically acceptable excipient and in particular a pharmaceutically acceptable carrier. These excipients include, inter alia, carriers (for example microcrystalline cellulose, lactose, mannitol), solvents (e.g. liquid polyethylene glycols), emulsifiers and dispersants or wetting agents (for example sodium dodecyl sulphate, polyoxysorbitan oleate), binders (for example polyvinylpyrrolidone), synthetic and natural polymers (for example albumin), stabilizers (e.g. antioxidants such as, for example, ascorbic acid), colours (e.g. inorganic pigments such as, for example, iron oxides) and masking flavours and/or odours.

The topical formulation comprising the *Harungana madagascariensis* leaf extract according to the disclosure may be in any physical form, for example in the form of an aqueous-alcoholic or aqueous-glycolic aqueous gel; a solution; a powder; a suspension, an emulsion, a microemulsion or a nanoemulsion, whether they are of or water-in-oil, oil-in-water, water-in-oil-in-water or oil-in-water-in-oil type.

The topical formulation comprising the *Harungana madagascariensis* leaf extract according to the disclosure may be packaged in a bottle, in a device of pump-"bottle" type, in pressurized form or in an aerosol device, in a device equipped with a perforated wall such as a grid or in a device equipped with a ball applicator (termed "roll-on").

The expression "topical" means that the composition according to the disclosure is used by application to the skin, the hair, the scalp or the mucus membranes, whether it is a direct application or an indirect application when the topical composition according to the invention is impregnated onto a support intended to be brought into contact with the skin (paper, wipe, textile, transdermal device, etc.).

Generally, the topical formulation comprising the *Harungana madagascariensis* leaf extract according to the disclosure also comprises chemical additives normally used in the field of formulations for topical use, such as foaming and/or detergent surfactants, thickening and/or gelling surfactants, thickeners and/or gelling agents, stabilizers, film-forming compounds, solvents and cosolvents, hydrotropic agents, spring or mineral waters, plasticizers, emulsifiers and co-emulsifiers, opacifiers, nacreous agents, overfatting agents, sequestrants, chelating agents, oils, waxes, antioxidants, fragrances, essential oils, preservatives, conditioning agents, deodorants, whitening agents intended for bleaching body hair and the skin, active ingredients intended to provide a treating and/or protective action with respect to the skin or the hair, sunscreens, inorganic fillers or pigments, particles which provide a visual effect or are intended for encapsulating active agents, exfoliant particles, texturing agents, optical brighteners, or insect repellants.

*Harungana madagascariensis* leaf extract can be further combined with any other suitable additive or pharmaceutically or cosmetically acceptable carrier. Such additives include any of the substances already mentioned, as well as any of those used conventionally, such as those described in Remington: The Science and Practice of Pharmacy (Gennaro and Gennaro, eds, 20th edition, Lippincott Williams & Wilkins, 2000); Theory and Practice of Industrial Pharmacy (Lachman et al., eds., 3rd edition, Lippincott Williams & Wilkins, 1986); Encyclopedia of Pharmaceutical Technology (Swarbrick and Boylan, eds., 2nd edition, Marcel Dekker, 2002). These can be referred to herein as "pharmaceutically or cosmetically acceptable carriers" to indicate they are combined with the active drug or compound and can be administered safely to a subject for therapeutic or cosmetic purposes.

As examples of foaming and/or detergent surfactants, optionally present in the topical formulation comprising the *Harungana madagascariensis* leaf extract according to the disclosure, mention may be made of anionic, cationic, amphoteric or nonionic foaming and/or detergent surfactants.

Among the anionic foaming and/or detergent surfactants that can be used in the topical formulation comprising the *Harungana madagascariensis* leaf extract according to the disclosure, mention may be made of alkali metal salts, alkaline earth metal salts, ammonium salts, amine salts or amino alcohol salts of alkyl ether sulfates, alkyl sulfates, alkylamido ether sulfates, alkylarylpolyether sulfates, monoglyceride sulfates, alpha-olefin sulfonates, paraffin sulfonates, alkyl phosphates, alkyl ether phosphates, alkyl sulfonates, alkylamide sulfonates, alkylaryl sulfonates, alkyl carboxylates, alkyl sulfosuccinates, alkyl ether sulfosuccinates, alkylamide sulfosuccinates, alkyl sulfoacetates, alkylsarcosinates, acylisethionates, N-acyltaurates, acyl lactylates, N-acylated derivatives of amino acids, N-acylated derivatives of peptides, N-acylated derivatives of proteins, or N-acylated derivatives of fatty acids.

Among the amphoteric foaming and/or detergent surfactants that can be used in the topical formulation comprising the *Harungana madagascariensis* leaf extract according to the disclosure, mention may be made of alkyl betaines, alkylamido betaines, sultaines, alkylamidoalkyl sulfobetaines, imidazoline derivatives, phosphobetaines, amphopolyacetates and amphopropionates.

Among the cationic foaming and/or detergent surfactants that can be used in the topical formulation comprising the *Harungana madagascariensis* leaf extract according to the disclosure, mention may particularly be made of quaternary ammonium derivatives.

Among the nonionic foaming and/or detergent surfactants that can be used in the topical formulation comprising the *Harungana madagascariensis* leaf extract according to the disclosure, mention may more particularly be made of alkyl polyglycosides comprising a linear or branched, and saturated or unsaturated aliphatic radical, comprising from 8 to 16 carbon atoms, such as octyl polyglucoside, decyl polyglucoside, undecylenyl polyglucoside, dodecyl polyglucoside, tetradecyl polyglucoside, hexadecyl polyglucoside, 1,12-dodecanediyl polyglucoside; ethoxylated derivatives of hydrogenated castor oil, such as the product sold under the INCI name "Peg-40 hydrogenated castor oil"; polysorbates such as Polysorbate 20, Polysorbate 40, Polysorbate 60, Polysorbate 70, Polysorbate 80 or Polysorbate 85; coconut amides; or N-alkylamines.

As examples of thickening and/or gelling surfactants optionally present in the topical formulation comprising the *Harungana madagascariensis* leaf extract according to the disclosure, mention may be made of optionally alkoxylated alkyl polyglucoside fatty esters, for instance ethoxylated methylpolyglucoside esters such as PEG 120 methyl glucose trioleate and PEG 120 methyl glucose dioleate sold respectively under the names Glucamate™ LT and Glumate™ DOE120; alkoxylated fatty esters such as PEG 150 pentaerythrytyl tetrastearate sold under the name Crothix™ DS53, PEG 55 propylene glycol oleate sold under the name Antil™ 141; fatty-chain polyalkylene glycol carbamates, for instance PPG-14 laureth isophoryl dicarbamate sold under the name Elfacos™ T211, or PPG-14 palmeth-60 hexyl dicarbamate sold under the name Elfacos™ GT2125.

As examples of thickeners and/or gelling agents optionally present in the topical formulation comprising the *Harungana madagascariensis* leaf extract according to the disclosure, mention may be made of linear or branched or crosslinked polymers of polyelectrolyte type, for instance acrylic acid homopolymer, methacrylic acid homopolymer, 2-methyl-[(1-oxo-2-propenyl)amino]-1-propanesulfonic acid (AMPS) homopolymer, copolymers of acrylic acid and of AMPS, copolymers of acrylamide and of AMPS, copolymers of vinylpyrolidone and of AMPS, copolymers of AMPS and of (2-hydroxyethyl) acrylate, copolymers of AMPS and of (2-hydroxyethyl) methacrylate, copolymers of AMPS and of hydroxyethylacrylamide, copolymers of AMPS and of N,N-dimethylacrylamide, copolymers of AMPS and of tris(hydroxyl-methyl)acrylamidomethane (THAM), copolymers of acrylic or methacrylic acid and of (2-hydroxyethyl) acrylate, copolymers of acrylic or methacrylic acid and of (2-hydroxyethyl) methacrylate, copolymers of acrylic or methacrylic acid and of hydroxyethylacrylamide, copolymers of acrylic or methacrylic acid and of THAM, copolymers of acrylic or methacrylic acid and of N,N-dimethylacrylamide, terpolymers of acrylic or methacrylic acid, of AMPS and of (2-hydroxyethyl) acrylate, terpolymers of acrylic or methacrylic acid, of AMPS and of (2-hydroxyethyl) methacrylate, terpolymers of acrylic or methacrylic acid, of AMPS and of THAM, terpolymers of acrylic or methacrylic acid, of AMPS and of N,N-dimethylacrylamide, terpolymers of acrylic or methacrylic acid, of AMPS and of acrylamide, copolymers of acrylic acid or methacrylic acid and of alkyl acrylates of which the carbon-based chain comprises between four and thirty carbon atoms, and more particularly between ten and thirty carbon atoms, copolymers of AMPS and of alkyl acrylates of which the carbon-based chain comprises between four and thirty carbon atoms and more particularly between ten and thirty carbon atoms. The linear or branched or crosslinked polymers of polyelectrolyte type optionally present in the topical composition according to the invention may be in the form of a solution, an aqueous suspension, a water-in-oil emulsion, an oil-in-water emulsion, or a powder. The linear or branched or crosslinked polymers of polyelectrolyte type optionally present in the topical composition according to the invention can be selected from the products sold under the names Simulgel™ EG, Simulgel™EPG, Sepigel™ 305, Simulgel™ 600, Simulgel™ NS, Simulgel™ INS 100, Simulgel™ FL, Simulgel™ A, Simulgel ™ SMS 88, Sepinov™EMT 10, Sepiplus™400, Sepiplus™265, Sepiplus™S, Aristoflex™AVC, Aristoflex™AVS, Novemer™EC-1, Flocare™ET 25, Flocare™ET 75, Flocare™ET 26, Flocare™ET 30, Flocare™ET 58, Flocare™PSD 30, Viscolam™AT 64, Viscolam™AT 100.

As examples of thickeners and/or gelling agents optionally present in the topical formulation comprising the *Harungana madagascariensis* leaf extract comprising the *Harungana madagascariensis* leaf extract according to the invention, mention may be made of polysaccharides consisting only of monosaccharides, such as glucans or glucose homopolymers, glucomannoglucans, xyloglycans, galactomannans of which the degree of substitution (DS) of the D-galactose units on the main chain of D-mannose is between 0 and 1, and more particularly between 1 and 0.25, such as galactomannans originating from cassia gum (DS = 1/5), from locust beam gum (DS = 1/4), from tara gum (DS = 1/3), from guar gum (DS = 1/2) or from fenugreek gum (DS = 1).

As examples of thickeners and/or gelling agents optionally present in the topical formulation comprising the *Harungana madagascariensis* leaf extract comprising the *Harungana madagascariensis* leaf extract according to the disclosure, mention may be made of polysaccharides consisting of monosaccharide derivatives, such as sulfated galactans and more particularly carrageenans and agar, uronans and more particularly algins, alginates and pectins, heteropolymers of monosaccharides and of uronic acids and more particularly xanthan gum, gellan gum, exudates of gum Arabic and of karaya gum, and glucosaminoglycans.

In the topical formulations comprising the *Harungana madagascariensis* leaf extract according to the disclosure also comprising at least one polysaccharide chosen from the group consisting of a galactomannan, gum Arabic and xanthan gum, the weight ratio between said polysaccharide and the anionic polyelectrolyte according to the invention is greater than or equal to 1/3 and less than or equal to 3/1, more particularly or equal to 1/2 and less than or equal to 3/2. The combination of the polysaccharide, as defined above, and of the anionic polyelectrolyte according to the invention makes it possible to obtain topical compositions according to the invention which are in the form of salt-rich emulsions, retaining a high viscosity and a uniform appearance after a prolonged storage period. Such topical compositions according to the invention can comprise, for 100% of their weight, from 0.1% to 25% by weight of at least one salt and can have a dynamic viscosity measured at 20°C, by means of a Brookfield viscometer, of greater than or equal to 30 000 mPa.s and less than or equal to 200 000 mPa.s.

As examples of thickeners and/or gelling agents optionally present in the topical formulation comprising the *Harungana madagascariensis* leaf extract according to the disclosure, mention may be made of cellulose, cellulose derivatives such as methylcellulose, ethylcellulose or hydroxypropylcellulose, silicates, starch, hydrophilic starch derivatives, and polyurethanes.

As examples of stabilizers optionally present in the topical formulation comprising the *Harungana madagascariensis* leaf extract according to the disclosure, mention may be made, for example, of microcrystalline waxes, and more particularly ozokerite, inorganic salts such as sodium chloride or magnesium chloride, and silicone polymers such as polysiloxane polyalkyl polyether copolymers.

As examples of solvents optionally present in the topical formulation comprising the *Harungana madagascariensis* leaf extract according to the disclosure, mention may be made of water, organic solvents such as glycerol, diglycerol, glycerol oligomers, ethylene glycol, propylene glycol, butylene glycol, 1,3-propanediol, 1,2-propanediol, hexylene glycol, diethylene glycol, xylitol, erythritol, sorbitol, water-soluble alcohols such as ethanol, isopropanol or butanol, and mixtures of water and said organic solvents.

As examples of spring or mineral waters optionally present in the topical formulation comprising the *Harungana madagascariensis* leaf extract according to the disclosure, mention may be made of spring or mineral waters having a mineralization of at least 300 mg/l, in particular Avene water, Vittel water, waters from the Vichy basin, Uriage water, Roche Posay water, Bourboule water, Enghien-les-bains water, Saint-Gervais-les bains water, Neris-les-bains water, Allevard-les-bains water, Digne water, Maizieres water, Neyrac-les-bains water, Lons le Saunier water, Rochefort water, Saint Christau water, Fumades water and Tercis-les-bains water.

As examples of hydrotropic agents optionally present in the topical formulation comprising the *Harungana madagascariensis* leaf extract according to the disclosure, mention may be made of the xylene sulfonates, cumene sulfonates, hexylpolyglucoside, 2-ethylhexylpolyglucoside and n-heptylpolyglucoside.

As examples of emulsifying surfactants optionally present in the topical formulation comprising the *Harungana madagascariensis* leaf extract according to the disclosure, mention may be made of nonionic surfactants, anionic surfactants and cationic surfactants.

As examples of nonionic emulsifying surfactants optionally present in the topical formulation comprising the *Harungana madagascariensis* leaf extract according to the disclosure, mention may be made of fatty acid esters of sorbitol, for instance the products sold under the names Montane™40, Montane™60, Montane™70, Montane™80 and Montane™85; compositions comprising glyceryl stearate and steric acid ethoxylated at between 5 mol and 150 mol of ethylene oxide, for instance the composition comprising steric acid ethoxylated at 135 mol of ethylene oxide and glyceryl stearate sold under the name Simulsol™ 165; mannitan esters; ethoxylated mannitan esters; sucrose esters; methylglucoside esters; alkyl polyglycosides comprising a linear or branched and saturated or unsaturated aliphatic radical, comprising from 14 to 36 carbon atoms, such as tetradecylpolyglucoside, hexadecylpolyglucoside, octadecylpolyglucoside, hexadecylpolyxyloside, octadecylpolyxyloside, eicosylpolyglucoside, dodecosylpolyglucoside, 2-octyldodecylpolyxyloside or 12-hydroxystearylpolyglucoside; compositions of linear or branched and saturated or unsaturated fatty alcohols, comprising from 14 to 36 carbon atoms, and of alkyl polyglycosides as described above.

As examples of nonionic surfactants optionally present in the topical formulation comprising the *Harungana madagascariensis* leaf extract according to the invention mention may be made of glyceryl stearate citrate, cetearyl sulfate, soaps such as sodium stearate or triethanolammonium stearate, and salified N-acylated amino acid derivatives, for instance stearoyl glutamate.

As examples of cationic emulsifier surfactants optionally present in the topical formulation comprising the *Harungana madagascariensis* leaf extract according to the disclosure, mention may be made of amine oxides, quaternium-82 and the surfactants described in patent application WO 96/00719 and mainly those in which the fatty chain comprises at least 16 carbon atoms.

As examples of opacifiers and/or nacreous agents optionally present in the topical formulation comprising the *Harungana madagascariensis* leaf extract according to the disclosure, mention may be made of sodium palmitate, sodium stearate, sodium hydroxystearate, magnesium palmitate, magnesium stearate, magnesium hydroxystearate, ethylene glycol monostearate, ethylene glycol distearate, polyethylene glycol monostearate, polyethylene glycol distearate, and fatty alcohols comprising from 12 to 22 carbon atoms.

As examples of texturing agents optionally present in the topical formulation comprising the *Harungana madagascariensis* leaf extract according to the disclosure mention may be made of N-acylated derivatives of amino acids, such as the lauroyl lysine sold under the name Aminohope™LL, the octenyl starch succinate sold under the name Dryflo™, the myristyl polyglucoside sold under the name Montanov™ 14, cellulose fibers, cotton fibers, chitosan fibers, talc, sericite and mica.

As examples of deodorants optionally present in the topical formulation comprising the *Harungana madagascariensis* leaf extract according to the disclosure, mention may be made, for example, of alkali metal silicates, zinc salts such as zinc sulfate, zinc gluconate, zinc chloride or zinc lactate; quaternary ammonium salts such as cetyltrimethylammonium salts, cetylpyridinium salts; glycerol derivatives such as glyceryl caprate, glyceryl caprylate, polyglyceryl caprate; 1,2-decanediol; 1,3-propanediol; salicylic acid; sodium bicarbonate; cyclodextrins; metal zeolites; Triclosan™; aluminum bromohydrate, aluminum chlorohydrates, aluminum chloride, aluminum sulfate, aluminum zirconium chlorohydrates, aluminum zirconium trichlorohydrate, aluminum zirconium tetrachlorohydrate, aluminum zirconium pentachlorohydrate, aluminum zirconium octochlorohydrate, aluminum sulfate, sodium aluminum lactate, complexes of aluminum chlorohydrate and glycol, such as the complex of aluminum chlorohydrate and of propylene glycol, the complex of aluminum dichlorohydrate and of propylene glycol, the complex of aluminum sesquichlorohydrate and of propylene glycol, the complex of aluminum chlorohydrate and of polyethylene glycol, the complex of aluminum dichlorohydrate and of polyethylene glycol, and the complex of aluminum sesquichlorohydrate and of polyethylene glycol.

As examples of oils optionally present in the topical formulation comprising the *Harungana madagascariensis* leaf extract according to the disclosure, mention may be made of mineral oils such as paraffin, liquid petroleum jelly, isoparaffins or white mineral oils; oils of animal origin, such as squalene or squalane; vegetable oils, such as phytosqualane, sweet almond oil, coconut oil, castor oil, jojoba oil, olive oil, rapeseed oil, peanut oil, sunflower oil, wheatgerm oil, corn germ oil, soybean oil, cottonseed oil, alfafa oil, poppyseed oil, pumpkin oil, evening primrose oil, millet oil, barley oil, rye oil, safflower oil, candlenut oil, passion flower oil, hazelnut oil, palm oil, shea butter, apricot kernel oil, beauty leaf oil, sysymbrium oil, avocado oil, calendula oil, oils derived from flowers or from vegetables, ethoxylated vegetable oils; synthetic oils, for instance fatty acid esters such as butyl myristate, propyl myristate, isopropyl myristate, cetyl myristate, isopropyl palmitate, octyl palmitate, butyl stearate, hexadecyl stearate, isopropyl stearate, octyl stearate, isocetyl stearate, dodecyl oleate, hexyl laurate, propylene glycol dicaprylate, lanolic acid-derived esters, such as isopropyl lanolate, isocetyl lanolate, fatty acid monoglycerides, diglycerides and triglycerides, for instance glyceryl triheptanoate, alkyl benzoates, hydrogenated oils, poly(alpha-olefin)s, polyolefins, for instance poly(isobutane), synthetic isoalkanes, for instance isohexadecane, isododecane, perfluoro oils; silicone oils, for instance dimethylpolysiloxanes, methylphenylpolysiloxanes, silicones modified with amines, silicones modified with fatty acids, silicones modified with alcohols, silicones modified with alcohols and fatty acids, silicones modified with polyether groups, epoxy-modified silicones, silicones modified with fluoro groups, cyclic silicones and silicones modified with alkyl groups. The term "oils" is intended to mean, in the present application, compounds and/or mixtures of compounds which are insoluble in water and which have a liquid aspect at a temperature of 25°C.

As examples of waxes optionally present in the topical formulation comprising the *Harungana madagascariensis* leaf extract according to the disclosure, mention may be made of beeswax, carnauba wax, candelilla wax, ouricury wax, Japan wax, cork fiber wax, sugarcane wax, paraffin waxes, lignite waxes, microcrystalline waxes, lanolin wax; ozokerite; polyethylene wax; silicone waxes; vegetable waxes; fatty alcohols and fatty acids which are solid at ambient temperature; glycerides which are solid at ambient temperature. The term "waxes" is intended to mean, in the present application, compounds and/or mixtures of compounds which are insoluble in water and which have a solid aspect at a temperature greater than or equal to 45°C.

As examples of active ingredients optionally present in the topical formulation comprising the *Harungana madagascariensis* leaf extract according to the disclosure, mention may be made of vitamins and derivatives thereof, in particular esters thereof, such as retinol (vitamin A) and esters thereof (retinyl palmitate for example), ascorbic acid (vitamin C) and esters thereof, sugar derivatives of ascorbic acid (for instance ascorbyl glucoside), tocopherol (vitamin E) and esters thereof (for instance tocopheryl acetate), vitamin B3 or B10 (niacinamide and derivatives thereof); compounds showing a skin-lightening or-depigmenteding action, for instance the ω-undecelynoyl phenylalanine sold under the name Sepiwhite™MSH, Sepicalm™VG, the glycerol monoester and/or diester of ω-undecelynoyl phenylalanine, ω-undecelynoyl dipeptides, arbutin, kojic acid, hydroquinone; compounds showing a soothing action, in particular Sepicalm™ S, allantoin and bisabolol; anti-inflammatories; compounds showing a moisturizing action, such as urea, hydroxyureas, glycerol, polyglycerols, glycerol glucoside, diglycerol glucoside, polyglyceryl glucosides, xylityl glucoside; polyphenol-rich plant extracts, for instance grape extracts, pine extracts, wine extracts, olive extracts; compounds showing a slimming or lipolytic action, for instance caffeine or derivatives thereof, Adiposlim™, Adipoless™, fucoxanthine; N-acylated proteins; N-acylated peptides, for instance Matrixil™; N-acylated amino acids; partial hydrolyzates of N-acylated proteins; amino acids; peptides; total protein hydrolyzates; soybean extracts, for example Raffermine™; wheat extracts, for example Tensine™ or Gliadine™; plant extracts, such as tannin-rich plant extracts, isoflavone-rich plant extracts or terpene-rich plant extracts; extracts of freshwater or seawater algae; marine plant extracts; marine extracts in general, such as corals; essential waxes; bacterial extracts; ceramides; phospholipids; compound showing an antimicrobial action or a purifying action, for instance Lipacide™ C8G, Lipacide™ UG, Sepicontrol™ A5; Octopirox™ or Sensiva™ SC50; compounds showing an energizing or stimulating property, for instance Physiogenyl™, panthenol and derivatives thereof, for instance Sepicap™ MP; anti-aging active agents, for instance Sepilift™ DPHP, Lipacide™ PVB, Sepivinol™, Sepivital™, Manoliva™, Phyto-Age™, Timecode™; Survicode™; anti-photoaging active agents; agents for protecting the integrity of the dermoepidermal junction; agents for increasing the synthesis of extracellular matrix components such as collagen, elastins, glycosaminoglycans; active agents which act favorably on chemical cell communication, for instance cytokines, or physical cell communication, for instance integrins; active agents which create a "heating" sensation on the skin, for instance skin microcirculation activators (for instance nicotinic acid derivatives) or products which create a feeling of "freshness" on the skin (for instance menthol and derivatives); active agents for improving skin microcirculation, for example veinotonics; draining active agents; active agents for decongestive purposes, for instance extracts of ginko biloba, of ivy, of horse chestnut, of bamboo, of ruscus, of butcher's broom, of *Centalla asiatica,* of fucus, of rosemary, of willow; agents for tanning or browning the skin, for instance dihydroxyacetone, isatin, alloxane, ninhydrin, glyceraldehyde, mesotartic aldehyde, glutaraldehyde, erythrulose.

As examples of antioxidants optionally present in the topical formulation comprising the *Harungana madagascariensis* leaf extract according to the disclosure, mention may be made of EDTA and salts thereof, citric acid, tartaric acid, oxalic acid, BHA (butylhydroxyanisol), BHT (butylhydroxytoluene), tocopherol derivatives such as tocopheryl acetate, mixtures of antioxidant compounds such as Dissolvine™ GL 47S sold by the company Akzo Nobel under the INCI name: Tetrasodium Glutamate Diacetate.

As examples of sunscreens optionally present in the topical formulation comprising the *Harungana madagascariensis* leaf extract according to the disclosure, mention may be made of those which appear in the modified cosmetics directive 76/768/EEC annex VII. Among the organic sunscreens optionally present in the topical composition according to the invention, mention may be made of the family of benzoic acid derivatives, for instance para-aminobenzoic acids (PABAs), in particular monoglyceryl esters of PABA, ethyl esters of N,N-propoxy PABA, ethyl esters of N,N-diethoxy PABA, ethyl esters of N,N-dimethyl-PABA, methyl esters of N,N-dimethyl-PABA, butyl esters of N,N-dimethyl-PABA; the family of anthranilic acid derivatives, for instance homomenthyl-N-acetyl anthranilate; the family of salicylic acid derivatives, for instance amyl salicylate, homomenthyl salicylate, ethylhexyl salicylate, phenyl salicylate, benzyl salicylate, or p-isopropylphenyl salicylate; the family of cinnamic acid derivatives, for instance ethylhexyl cinnamate, ethyl -4-isopropyl cinnamate, methyl 2,5-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano-β-phenylcinnamate, mono(2-ethylhexanoyl)glyceryl di(para-methoxycinnamate); the family of benzophenone derivatives, for instance 2,4-dihydroxybenzophenone, 2,2'-dihydroxy-4-methoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octyloxybenzophenone, 4-hydroxy-3-carboxybenzophenone; 3-(4'-methylbenzylidene)-d,l-camphor, 3-(benzylidene)-d,l-camphor, camphor benzalkonium methosulfate; urocanic acid, ethyl urocanate; the family of sulfonic acid derivatives, for instance 2-phenylbenzimidazole-5 sulfonic acid and salts thereof; the family of triazine derivatives, for instance hydroxyphenyl triazine, ethylhexyloxyhydroxyphenyl-4-methoxyphenyltriazine, 2,4,6-trianillino-(p-carbo-2'-ethylhexyl-1'-oxy)-1,3,5-triazine, benzoic acid 4,4-((6-(((1,1-dimethylethyl)amino)carbonyl)phenyl)amino)-1,3,5-triazine-2,4-diyl diimino) bis(2-ethylhexyl) ester, 2-phenyl-5-methylbenzoxazole, 2,2'-hydroxy-5-methylphenylbenzotriazole, 2-(2'-hydroxy-5'-t-octylphenyl)benzotriazole, 2-(2'-hydroxy-5'-methyphenyl)benzotriazole; dibenzazine; dianisoylmethane, 4-methoxy-4"-t-butylbenzoylmethane; 5-(3,3-dimethyl-2-norbornylidene)-3-pentan-2-one; the family of diphenyl acrylate derivatives, for instance 2-ethylhexyl-2-cyano-3,3-diphenyl-2-propenoate, ethyl-2-cyano-3,3-diphenyl-2-propenoate; the family of polysiloxanes, for instance benzylidene siloxane malonate.

Among the inorganic sunscreens, also known as "inorganic screens", optionally present in the topical formulation comprising the *Harungana madagascariensis* leaf extract according to the disclosure, mention may be made of titanium oxide, zinc oxide, cerium oxide, zirconium oxide, yellow, red or black iron oxides, and chromium oxides. These inorganic screens may or may not be micronized, may or may not have undergone surface treatments and may be optionally present in the form of aqueous or oil predispersions.

As examples of active ingredients optionally present in the topical formulation comprising the *Harungana madagascariensis* leaf extract according to the disclosure, mention may be made of active ingredients used as anti-acne agents such as antibiotic agents, anti-bacteria agents, and for example active agents from the family of licosamids and more particularly clydamicine, active agents from the family of macrolides and more particularly erythromycin.

As examples of active ingredients optionally present in the topical formulation comprising the *Harungana madagascariensis* leaf extract according to the disclosure, mention may be made of active ingredients used as antimicrobian agents such as azelic acid and/or benzoyl peroxide, retinoids such as for example trenoid and its derivatives such as isotreoid, adapalene, minerals such as zinc sulfate, copper sulfate and sulfur.

As examples of active ingredients optionally present in the topical formulation comprising the *Harungana madagascariensis* leaf extract according to the disclosure, mention may be made of active ingredients used anti-acne and/or antiseborrheic agents such as lithium, lithium gluconate, sulfur selenium, cade oil, red algae extracts such as for example extract of *Achrocaetium Moniliforme* commercialized under the trade-name of CONTACTICELL™, the halophyte plant extract of *Chritmum maritimum* commercialized under the trade-name of Native Essence™.

The dosage of a pharmaceutical composition or cosmetic composition comprising *Harungana madagascariensis* leaf extract according to the present disclosure can be generally selected with reference to the type of disease or disorder and/or the disease or disorder status in order to provide the desired therapeutic or cosmetic activity. These amounts can be determined routinely for a particular patient or person to be cosmetically treated, where various parameters are utilized to select the appropriate dosage (e.g., type of disease or disorder, age of patient, disease or disorder status, patient health, weight, etc.), or the amounts can be relatively standard and can be easily determined by a person skilled in the art.

The amount of the administered *Harungana madagascariensis* leaf extract can vary widely according to such considerations as the particular compound and dosage unit employed, the mode and time of administration, the period of treatment, the age, sex, and general condition of the patient or person to be treated, the nature and extent of the condition treated, the rate of drug or compound metabolism and excretion, the potential drug combinations and drug-drug interactions, and the like.

The present invention will be further described with reference to the following aspects and embodiments. They can be combined freely unless the context clearly indicates otherwise.

In one embodiment, the acne is acne vulgaris and/or juvenile acne vulgaris. The term "juvenile acne vulgaris" is meant to denote acne vulgaris in patients up to and including 21 years of age, preferably up to and including 18 years of age.

In another embodiment, the extract is provided in a formulation at a concentration of ≥ 0.005 weight % to ≤ 0.25 weight % (based on the weight of the dry extract and the total weight of the formulation). Preferably, the concentration is ≥ 0.05 weight % to ≤ 0.15 weight %, more preferred ≥ 0.07 weight % to ≤ 0.1 weight %. This formulation may be prepared from a stock solution of the extract in a glycerine/water mixture by further diluting the stock solution in a liquid or carrier phase. For example, a solution of 5% (weight/volume) of *Harungana madagascariensis* leaf extract in glycerol/water (60/40 by weight) may be used as a stock solution.

It may nevertheless be necessary where appropriate to deviate from the stated amounts; in particular as a function of the bodyweight, route of administration, individual response to the active ingredient, nature of the preparation and time or interval over which administration takes place. Thus, it may be sufficient in some cases to make do with less than the aforementioned minimum amount, whereas in other cases the stated upper limit must be exceeded. It may in the event of administration of larger amounts be advisable to divide these into a plurality of individual doses over the day.

In another embodiment, the acne is at least partially caused by Propionibacterium acnes infection.

In another embodiment, the extract has a weight % content of polyphenolic compounds as assayed by the Folin-Ciocalteu colorimetric method greater than of equal to (≥) 10 weight % (based on the dry leaf extract). Preferably, said content is ≥ 10 weight% and less than or equal to (≤) 50 weight %. More preferably, said content is ≥ 20 weight % and ≤ 40 weight %.

Preferably, said polyphenolic compounds comprise flavonoids selected from the group of quercitrin, quercetin, glycosylated quercetin, catechin, taxifoline, taxifoline rhamnoside, taxifoline arabinoside, isorhamnetin and/or astilbine.

In another particular embodiment, the weight/volume % content of anthraquinone compounds in said *Harungana madagascariensis* leaf extract is ≤ 0.03%, as determined by HPLC-MS from a 5% solution (weight/volume) of the extract in glycerol and water (60:40 weight/weight). Preferably weight/volume % content of anthraquinone compounds in said *Harungana madagascariensis* leaf extract is ≤ 0.02% (w/v), and more preferably ≤ 0.01% (w/v) of. These anthraquinones are believed to exhibit detrimental effects and should be avoided, even if only for reasons of caution.

Examples for anthraquinones to be excluded as much as it is technically possible are: chrysophanol, hypericine, alizarin, rubiadin, nordamnacanthal, moridone, lucidin, lucidin-3-primveroside, morindone-6-proveroside, 2-methyl 3,5,6-trihydroxy-anthraquinone, 3-hydroxy-morindone, 5,6-dihydroxy-lucidin, 2-methyl-3,5,6-trihydroxy-anthraquinone-6-primveroside, 3-hydroxy-morindone-6-primveroside, 5,6-dihydroxy-lucidin-3-primveroside, lucidin-ethylether, pseudopurpurin, purpurin, ruberythric acid, anthragallol-1,2-dimethylether, anthragallol-1,3-dimethylether, 1-hydroxy-2-hydroxymethyl-anthraquinone, 1-hydroxy-2-hydroxy-anthraquinone, 2-hydroxy-1,3,4-trimethoxy-anthraquinone, 4-methoxy-1,3,5-trihydroxy-anthraquinone, 1,3-dihydroxy-4-methoxy-anthraquinone, 1,3-dihydroxy-2,5-dimethoxy-anthraquinone, physcion, emodin, purpurin carboxylic acid glycoside, galiosin, islandin, xanthorin, obtusifolin, aloe-emodin, aurantio-obtusin, alizarin-1-methylether and 1,8-dihydroxy-anthraquinone. In another particular embodiment, the composition as hereinbefore defined, further comprises one or more topically acceptable excipients and/or one or topically acceptable active ingredients.

The present disclosure, also relates a method of preparing *Harungana madagascariensis* leaf extract comprising the steps of:
A) Extracting dry and crushed *Harungana madagascariensis* leaves with a solvent mixture comprising water and ethanol, thereby obtaining a crude extract;
B) Filtering said crude extract, thereby obtaining a crude extract filtrate;
**characterized in that** the method further comprises the steps:
C) Contacting said crude extract filtrate with activated charcoal, thereby obtaining a composition;
D) Filtering said composition obtained after step C), thereby obtaining an extract filtrate;
E) Concentrating said extract filtrate, thereby obtaining said *Harungana madagascariensis* leaf extract.

Without wishing to be bound to theory it is believed that the step of contacting the crude extract with activated charcoal reduces the amount of anthraquinone compounds present in the composition.

In one aspect of the method according to the disclosure, the *Harungana madagascariensis* leaf extract obtained after step E) comprises ≤ 0.03% (weight/volume) of anthraquinone compounds, as determined by HPLC-MS from a 5% solution (weight/volume) of the extract in glycerol and water (60:40 weight/weight).

Preferably, the content is ≤ 0.02 % (w/v), more preferred ≤ 0.01% (w/v).

These anthraquinones are believed to exhibit detrimental effects and should be avoided, even if only for reasons of caution. Examples for anthraquinones to be excluded as much as it is technically possible are: chrysophanol, hypericine, alizarin, rubiadin, nordamnacanthal, moridone, lucidin, lucidin-3-primveroside, morindone-6-proveroside, 2-methyl-3,5,6-trihydroxy-anthraquinone, 3-hydroxy-morindone, 5,6-dihydroxy-lucidin, 2-methyl-3,5,6-trihydroxy-anthraquinone-6-primveroside, 3-hydroxy-morindone-6-primveroside, 5,6-dihydroxy-lucidin-3-primveroside, lucidin-ethylether, pseudopurpurin, purpurin, ruberythric acid, anthragallol-1,2-dimethylether, anthragallol-1,3-dimethylether, 1-hydroxy-2-hydroxymethyl-anthraquinone, 1-hydroxy-2-hydroxyanthraquinone, 2-hydroxy-1,3,4-trimethoxy-anthraquinone, 4-methoxy-1,3,5-trihydroxy-anthraquinone, 1,3-dihydroxy-4-methoxy-anthraquinone, 1,3-dihydroxy-2,5-dimethoxy-anthraquinone, physcion, emodin, purpurin carboxylic acid glycoside, galiosin, islandin, xanthorin, obtusifolin, aloe-emodin, aurantio-obtusin, alizarin-1-methylether and 1,8-dihydroxy-anthraquinone.

Another aspect of the present invention is the *Harungana madagascariensis* leaf extract for use to treat and/or to prevent acne, in a method of treatment of the human body by therapy.

It may be pointed out that in one embodiment of this use, the acne is acne vulgaris and/or juvenile acne vulgaris.

In another embodiment of this use, the extract has a content of polyphenolic compounds as assayed by the Folin-Ciocalteu colorimetric method of ≥ 10 weight % (based on the dry leaf extract). Preferably, the content is ≥ 10 weight% to ≤ 25 weight %, more preferred ≥ 15 weight % to ≤ 20 weight %.

In another embodiment of this use, the extract comprises ≤ 0.03% (weight/volume) of anthraquinone compounds, as determined by HPLC-MS from a 5% solution (weight/volume) of the extract in glycerol and water (60:40 weight/weight). Preferably, the content is ≤ 0.02 % (w/v), more preferred ≤ 0.01% (w/v%).

The disclosure also relates to the use of the composition as hereinbefore defined, in cosmetic.

The present invention will be further described with reference to the following figures and examples without wishing to be limited by them.
- Figure 1 shows experimental data from example 2;
- Figure 2 shows experimental data from example 2 in a semi-logarithmic plot;
- Figure 3 shows experimental data from example 3.

### Example 1: extraction process

Crushed dry leaves of *Harungana madagascariensis* Lam. ex Poir. were extracted with a mixture of ethanol and water 50:50 (v/v). The solution was stirred and heated below 60 °C during 1 hour. After plant removal, ethanol extract was decolorized on charcoal. After filtration the solution was concentrated to obtain an aqueous solution. After settling during 12 hours at 4 °C, the solution was filtered.

### Version A : dry extract

After settling, extract was filtered and was finally freeze-dried to get a powder form.

### Version B: liquid extract / 5% (w/v) solution

The same protocol was applied with changing the last step to obtain a liquid form. After settling filtration, adjustment was possible by adding glycerol to targeted a 5% (w/v) extract in a water-glycerin blend 40/60 (v/v).

The final extract was characterized by dry matter content, high performance liquid chromatography (HPLC) and spectrophotometry for polyphenols assay with a content determined to be more than 20% (on dry extract). The final composition showed, using liquid chromatography, the presence of numerous flavonoids such as quercitrin, quercetin glycosylated, catechin, taxifolines rhamnoside and arabinoside, isorhamnetin and astilbine.
In the following examples 2-5 the *Harungana madagascariensis* (HMA) extract used was prepared in accordance with example 1, version B.

### Example 2: antibacterial properties of HMA extract against Propionibacterium acnes

A study was conducted to examine the effect of HMA extract on Propionibacterium acnes bacteria. Extract concentrations were 0.1% (v/v), 0.5% (v/v) and 1.0% (v/v). Furthermore, a blank sample containing no extract was used.

In the study, 10 ml of a solution of the extract in sterile purified water with the respective concentration were introduced into a sterile tube. 0.1 ml of an inoculation solution containing ca. 10⁵ colony forming units of Propionibacterium acnes were added. The sample was homogenized and kept at a temperature of 37 °C ± 1 °C. The amount of microorganisms present was determined according to Ph. Eur. 2.6.12 after 0, 4, 8, 24 and 48 hours.

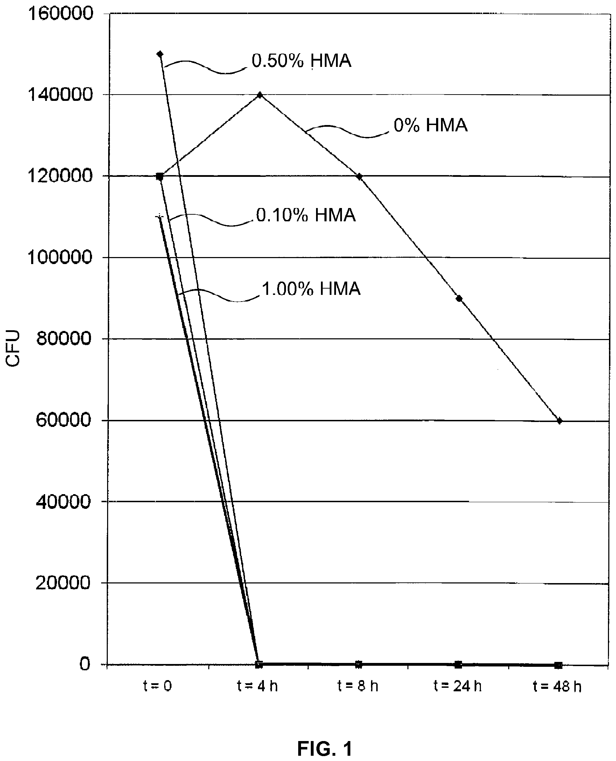

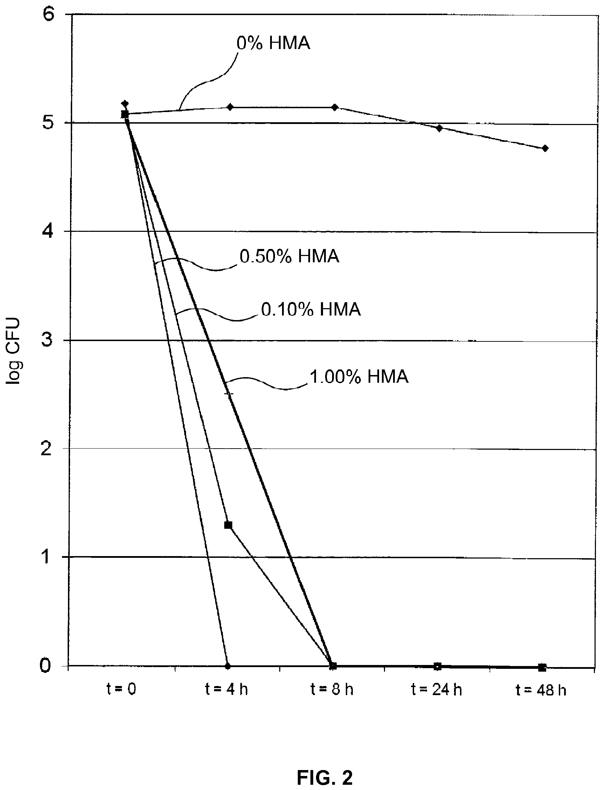

The results are shown in FIG. 1 and FIG. 2. FIG. 1 is a plot of the number of colony forming units (CFU, y-axis) against the time. Designations such as "0.50% HMA" identify the amount of *Harungana madagascariensis* (HMA) extract associated with the individual plot; in this instance it is the sample with 0.5 % (v/v) HMA extract. FIG. 2 is a semi-logarithmic plot of the experimental data as already shown in FIG. 1. The antibacterial effect of HMA extract against Propionibacterium acnes is readily apparent.

### Example 3: effect of HMA extract on lipase activity

In the present example, the effect of HMA extract was evaluated on the lipase activity with a biochemical test using a radioactive substrate. The lipase catalyzes the hydrolysis of glycerol-ester (glycerides) and long chain fatty acid emulsions. The substrate of this enzyme is not a simple molecule but a non-aqueous phase of aggregated lipids. The usual method to quantify the lipase activity is titration (pH variation) which is not often efficient for the testing of inhibitory compounds and generates several artifacts. An alternative and more efficient method (followed in the present example) was to use a radioactive complex lipid substrate.

The quantification of lipase activity was undertaken as follows:
Samples of HMA extract to be analyzed were prepared from a stock solution containing 5% (w/v) in glycerol/ultra-pure water (60/40).

The compound and the reference (Orlistat tested at 1000 nM) were incubated for 2 hours at 37 °C in presence of the enzyme, pancreatic lipase (PL; 3.1.1.3) at 125 U/ml and the radioactive substrate (3.34 µCi/tube, stable emulsion of triolein at 5.25 mM corresponding to a mix of cold and titriated triolein). A negative control was performed by incubating the substrate alone, without enzyme in the reaction mix, in order to determine the background signal.

After incubation, the reaction was stopped by adding a free fatty acid extraction solution. The extraction was then carried out by several steps of vortex and centrifugation. The superior phase (fatty acid containing fraction) was collected and the liquid scintillator was added for the counting.

All experimental conditions were performed in n=3, except for control condition in n=6.

Quenching controls were performed in parallel in n=1. These quenching conditions are control conditions in which the test compound was added at the end of the incubation time, just before the fatty acid extraction.

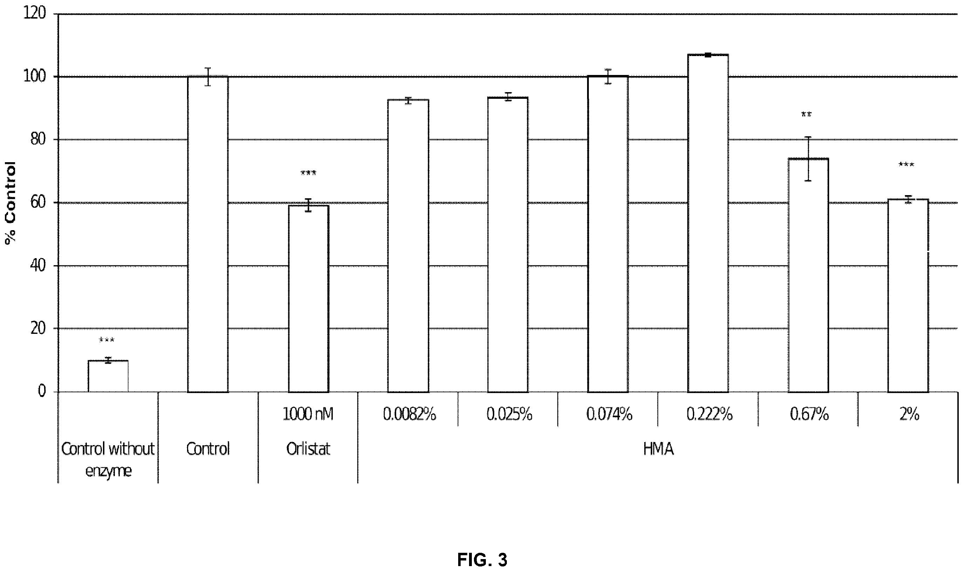

Raw data were analyzed using Microsoft Excel® software. The inter-group comparisons were performed by an unpaired Student's t-test.
The following table summarizes the results which are also depicted in Figure 3:

| **Test compound** | **Conc. [w/v]** | **% Inhibition** | **% sem** | ***p*** |
|---|---|---|---|---|
| Control | - | - | - | - |
| Control without enzyme | - | 0 | 3 | - |
| Orlistat | 1000 nM | 41 | 2 | *** |
| HMA | 0.0082% | 7 | 1 | *ns* |
| HMA | 0.025% | 6 | 1 | *ns* |
| HMA | 0.074% | 0 | 2 | *ns* |
| HMA | 0.222% | -7 | 1 | *ns* |
| HMA | 0.67% | 26 | 7 | ** |
| HMA | 2% | 39 | 1 | *** |

| | | | | |
|---|---|---|---|---|
| sem: standard error of the mean; *p*: threshold for statistical significance: *ns*: > 0.05 (not significant); *: 0.01 to 0.05 (significant); **: 0.001 to 0.01 (very significant); ***: < 0.001 (extremely significant) | | | | |

The reference, Orlistat, tested at 1000 nM, significantly decreased the activity of the lipase (41% of inhibition). This effect was expected and validated the assay. Under the experimental conditions of the assay, HMA extract, tested at 0.67% and 2%, had a concentration-dependent inhibitory effect on the lipase activity (26% and 39% of inhibition, respectively). At the lower concentrations, this compound had no effect.

### Example 4: anti-inflammatory effect of HMA

In the present example, the potential anti-inflammatory effect of HMA extract was investigated in human peripheral blood mononuclear cells (PBMCs) under stimulated conditions. More precisely, the effect of the test compound was evaluated on IL-8, IL-10 and IL-12 release by LPS-stimulated PBMCs using specific ELISA kits.

The stimulation of PBMCs by LPS (lipopolysaccharide), a highly potent activator of the innate immune system and a major marker for the host's recognition of intruding Gram-negative pathogens, leads to the release of a large spectrum of inflammatory mediators that are essential for early innate and later adaptive immune response.

The quantification of anti-inflammatory effects was undertaken as follows: Samples of HMA extract to be analyzed were prepared from a stock solution containing 5% (w/v) in glycerol/ultra-pure water (60/40).

PBMCs were isolated from whole blood, seeded in 96-well plates and incubated in culture medium for 1 hour. The medium was then replaced by culture medium containing or not (stimulated control) the test compound or the reference compound, i.e., dexamethasone tested at 10⁻⁷ M, and cells were pre-incubated for 1 hour. The inflammatory inducer, LPS (1 µg/ml) was then added and cells were incubated for 24 hours. A non-stimulated control was also performed in parallel. All experimental conditions were performed in n=3.

After incubation, the culture supernatants were collected in order to quantify IL-8, IL-10 and IL-12 release. A viability assay was performed on cells according to a standard alamarBlue® protocol.

The quantity of IL-8, IL-10 and IL-12 released in the culture supernatants was measured using specific ELISA kits according to the supplier's instructions.

After treatment, the cells were incubated with alamarBlue® reduced in resorufin via the mitochondrial reduction reactions of metabolically active cells. This transformation is proportional to the number of living cells. The optical density (OD) at 570nm was recorded using a microplate reader (VERSAmax, Molecular Devices).

Raw data were analyzed using Microsoft Excel® software. The inter-group comparisons were performed by an unpaired Student's t-test.

The following table shows the effect of HMA extract on IL-8 release by LPS-stimulated PBMCs:

| **Compound** | **Conc. [w/v]** | **% Inhibition** | **% sem** | ***p*** | **Viability % stimulated control** |
|---|---|---|---|---|---|
| control (non-stimulated) | - | 100 | 0 | *** | 102 |
| Control | - | 0 | 8 | - | 100 |
| Dexamethasone | 10⁻⁷ M | 72 | 2 | ** | 88 |
| HMA | 0.0137% | 5 | 11 | *ns* | 99 |
| HMA | 0.041% | 9 | 7 | *ns* | 94 |
| HMA | 0.123% | 5 | 2 | *ns* | 99 |

| | | | | | |
|---|---|---|---|---|---|
| sem: standard error of the mean; *p*: threshold for statistical significance: *ns*: > 0.05 (not significant); *: 0.01 to 0.05 (significant); **: 0.001 to 0.01 (very significant); ***: < 0.001 (extremely significant) | | | | | |

The stimulation of PBMCs by LPS (1 µg/ml) for 24 hours resulted in a strong release of IL-8 (-12823 pg/ml). Dexamethasone, tested at 10⁻⁷ M, clearly inhibited LPS-induced IL-8 release (72% of inhibition). These results were expected and validated the assay. HMA extract, tested at 0.0137, 0.041 and 0.123% (w/v), showed no significant effect on IL-8 release. The following table shows the effect of HMA extract on IL-10 release by LPS-stimulated PBMCs:

| **Compound** | **Conc. [w/v]** | **% Inhibition (I) or % stimulation (S)** | **%sem** | ***p*** | **Viability % stimulated control** |
|---|---|---|---|---|---|
| control (non-stimulated) | - | 100 (I) | 0 | *** | 102 |
| control | - | 0 (I) | 3 | - | 100 |
| Dexamethasone | 10⁻⁷ M | 32 (I) | 2 | *** | 88 |
| HMA | 0.0137% | -13 (S) | 5 | *ns* | 99 |
| HMA | 0.041% | 0 (S) | 5 | *ns* | 94 |
| HMA | 0.123% | 157 (S) | 5 | *** | 99 |

| | | | | | |
|---|---|---|---|---|---|
| sem: standard error of the mean; *p*: threshold for statistical significance: *ns*: > 0.05 (not significant); *: 0.01 to 0.05 (significant); **: 0.001 to 0.01 (very significant); ***: < 0.001 (extremely significant) | | | | | |

The stimulation of PBMCs by LPS (1 µg/ml) for 24 hours resulted in a marked release of IL-10 (478 pg/ml). Dexamethasone, tested at 10⁻⁷ M, showed a potent inhibitory effect on LPS-induced IL-10 release (32% of inhibition). These results were expected and validated the assay. HMA extract, tested at 0.123% (w/v), strongly stimulated IL-10 release by the PBMCs (stimulation of 157%). At the lower concentrations, this compound did not modulate the release of IL-10.

The following table shows the effect of HMA extract on IL-12 release by LPS-stimulated PBMCs:

| **Compound** | **Conc. [w/v]** | **% Inhibition** | **% sem** | ***p*** | **Viability % stimulated control** |
|---|---|---|---|---|---|
| Control (non-stimulated) | - | 100 | 0 | ** | 102 |
| Control | - | 0 | 16 | - | 100 |
| Dexamethasone | 10⁻⁷ M | >100 | 0 | ** | 88 |
| HMA | 0.0137% | -17 | 9 | *ns* | 99 |
| HMA | 0.041% | 23 | 5 | *ns* | 94 |
| HMA | 0.123% | 93 | 2 | ** | 99 |

| | | | | | |
|---|---|---|---|---|---|
| sem: standard error of the mean; *p*: threshold for statistical significance: *ns*: > 0.05 (not significant); *: 0.01 to 0.05 (significant); **: 0.001 to 0.01 (very significant); ***: < 0.001 (extremely significant) | | | | | |

The stimulation of PBMCs by LPS (1 µg/ml) for 24 hours, resulted in a strong release of IL-12 (1429 pg/ml), which was totally repressed by the reference dexamethasone, tested at 10⁻⁷ M (100% of inhibition). These results were expected and validated the assay. HMA extract, tested at 0.123% (w/v) had a strong inhibitory effect on LPS-induced IL-12 release (93% of inhibition). At the lower concentrations, the compound had no clear effect.

### Example 5: anti-oxidant capacity of HMA

In the present example, the effect of HMA extract was evaluated on oxidative stress. More specifically, its protective effect against lipid peroxidation (acellular reaction) was evaluated by measuring the production of thiobarbituric acid reactive substances (TBARS).

The quantification of anti-oxidant capacity was undertaken as follows:
Samples of HMA extract to be analyzed were prepared from a stock solution containing 5% (w/v) in glycerol/ultra-pure water (60/40).

Phosphatidylcholine micelles were incubated in presence of hemin (lipid peroxidation inducer, 5 µM) with or without (control) the test compound or the reference compound (antioxidant, Trolox (6-hydroxy-2,5,7,8-tetramethylchroman-2-carboxylic acid), 10 µM) for 1 hour at 37 °C. Additional control conditions were also included in the experimental design: without inducer (non-activated condition), 'Interference' controls (test compound added to the reactive medium without micelles of lipids to assess a potential auto-fluorescence of compounds) and 'Quenching' controls (test compound added at the end of reaction to check for potential quenching of the signal directly by the test compounds). All experimental conditions were performed in n=3, except the 'Interference' and 'Quenching' controls performed in n=2.

At the end of the incubation, the oxidation of lipids was stopped by addition of indole. This reaction medium obtained after lipid peroxidation was added to a mix of thiobarbituric acid (TBA), trichloracetic acid (TCA) and butylhydroxytoluene (BHT, a strong antioxidant to protect from any lipid peroxidation phenomena in this part of the reaction). Lipids were then removed from the mix using a chloroform separation step. TBARS were then produced by heating the aqueous phase at 100 °C for 20 minutes, thus allowing malondialdehyde (MDA lipid peroxidation by-product) adduct formation with TBA. TBARS thus produced were then evaluated by fluorescence measurement (λex 540 nm; λem 590 nm).

The results are expressed as TBARS equivalent, representing the antioxidant activity expressed as Trolox equivalent of the test compound.

Raw data were analyzed using Microsoft Excel® software. The inter-group comparisons were performed by an unpaired Student's t-test.

The following table shows the effect of HMA extract on TBARS production:

| **Compound** | **Conc. [w/v]** | **% Inhibition** | **% sem** | ***p*** |
|---|---|---|---|---|
| Control(non-activated) | - | 100 | 1 | *** |
| Control | - | 0 | 2 | - |
| Trolox | 10 µM | 72 | 1 | *** |
| HMA | 0.0002% | 46 | 3 | *** |
| HMA | 0.0006% | 75 | 1 | *** |
| HMA | 0.00185% | 75 | 3 | *** |
| HMA | 0.0055% | 75 | 0 | *** |
| HMA | 0.016% | 75 | 1 | *** |
| HMA | 0.05% | 75 | 1 | *** |

| | | | | |
|---|---|---|---|---|
| sem: standard error of the mean; *p*: threshold for statistical significance: *ns*: > 0.05 (not significant); *: 0.01 to 0.05 (significant); **: 0.001 to 0.01 (very significant); ***: < 0.001 (extremely significant) | | | | |

Hemin(5µM) largely induced the lipid peroxidation reaction by markedly stimulating the production of TBARS. The reaction caused by hemin was strongly inhibited by the reference Trolox, tested at 10 µM (72% of inhibition). These results were expected and validated the assay.

HMA extract, tested from 0.0002 to 0.05%, strongly inhibited a TBARS production, with a maximum effect (75%) as early as 0.0006%. Under the experimental conditions of this example, compound HMA exhibited anti-oxidant effect when evaluated in an acellular assay of lipid peroxidation reaction measuring TBARS production.

### Example 6: anti-acne properties of HMA in adults (double blind test)

In the context of the following examples, DX represents day *X* of the respective study/test. After twice daily use for 28 days, an emulsion containing 2% (w/w) of HMA extract:
- Induced a decrease of the number of acne lesions characterized by a significant decrease of the number of microcysts on D28 (-2.3±4.0, p=0.0034),
- Induced an improvement of the skin state in terms of lesions on D28 characterized by:
   - A significant improvement of the lesions number score (-0.5±1.0, p=0.0091);

   - A significant improvement of the lesions visibility score (-0.8±1.7, p=0.0116);
   - A significant improvement of the lesions color intensity score (-0.9±1.8, p=0.0084);
   - A significant improvement of the purifying effect score (-0.9±1.5, p=0.0002);
- Induced an improvement of the skin state in terms of complexion on D28 characterized by:
   - A significant improvement of the stains score (1.0±1.4, p=0.0005);
   - A significant improvement of the redness score (0.8±1.7, p=0.0181);
   - A significant improvement of the pores score (0.9±1.4, p=0.0006);
   - A significant improvement of the roughness score (0.9±1.5, p=0.0016);
- Induced a seboregulating effect characterized by a significant decrease of the sebum rate on D28 (-28.7±46.4, p=0.002),
- Induced a significant decrease of the cutaneous microcirculation on D3 (-17.8±3.1, p<.0001), D7 (-15.3±3.4, p<.0001) and D28 (-20.7±4.1, p<.0001),
- Did not induce any noticeable effect on the complexion radiance;
- Was appreciated by the subjects for its properties and for its efficacy.

Consequently, in comparison to the initial state, the product containing HMA extract presented an anti-acne and purifying efficacy which are confirmed by the decrease of the number of the microcysts and improvements of the clinical scores of lesions (number, visibility, color intensity, purifying effect). The product induced the improvements of the clinical scores of complexion (stains, redness, pores and roughness) thus improved the uniformity of complexion. The anti-inflammatory efficacy was confirmed by the decrease of cutaneous microcirculation and the decrease of the sebum rate characterized a seboregulating efficacy of the tested product.

After twice daily use for 28 days, a comparison emulsion without 2% HMA extract:
- Induced a decrease of the number of acne lesions characterized by:
   - A significant decrease of the number of microcysts on D28 (-4.5±5.4, p=0.0001);
   - A significant decrease of the number of papules on D28 (-2.0±2.8, p=0.0002);
   - A significant decrease of the number of pustules on D28 (-0.6±0.9, p=0.0058);
- Induced an improvement of the skin state in terms of lesions on D28 characterized by:
   - A significant improvement of the lesions number score (-1.0±0.9, p<0.0001);
   - A significant improvement of the lesions visibility score (-1.9±1.8, p<0.0001);
   - A significant improvement of the lesions color intensity score (-1.5±1.8, p=0.0001),
   - A significant improvement of the lesions size score (-1.4±19, p=0.0005);
   - A significant improvement of the purifying effect score (-1.8±1.6, p<0.0001);
- Induced an improvement of the skin state in terms of complexion on D28 characterized by:
   - A significant improvement of the stains score (1±1.7, p<0.0034),
   - A significant improvement of the redness score (1.2±2.0, p=0.0036),
   - A significant improvement of the pores score (1.3±1.4, p<0.0001),
   - A significant improvement of the roughness score (1.4±2.1, p=0.0003),
- Induced a seboregulating effect characterized by a significant decrease of the sebum rate on D28 (-33.7±41.5, p=0.0002),
- Induced a significant decrease of the cutaneous microcirculation on D3 (-8.0±3.3, p=0.0196), D7 (-13.5±3.5, p=0.0003) and D28 (-16.3±4.3, p=0.0003),
- Did not induce any noticeable effect on the complexion radiance,
- Was appreciated by the subjects for its properties and for its efficacy.

Consequently, in comparison to the initial state, the comparison product presented an anti-acne and purifying efficacy which are confirmed by the decrease of the number of the microcysts, papules and pustules and by improvements of the clinical scores of lesions (number, visibility, color intensity, size, purifying effect). The product induced the improvements of the clinical scores of complexion (stains, redness, pores and roughness) thus improved the uniformity of complexion. The anti-inflammatory efficacy was confirmed by the decrease of cutaneous microcirculation and the decrease of the sebum rate characterized a seboregulating efficacy of the tested product.

Comparison of the products revealed that the anti-acne and purifying efficacy of the comparison product (in terms of number of papules and clinical scores concerning purifying effect, number of lesions, their visibility and size) was stronger than for the product containing HMA.

However, the anti-inflammatory efficacy of the product containing HMA (on D3 only) was stronger than for the comparison product.

No relevant adverse reaction was observed during the study.

### Example 7: anti-acne properties of HMA in juveniles (double blind test)

After twice daily use for 28 days, an emulsion containing 2% (w/v) of HMA extract:
- Induced a decrease of the number of acne lesions characterized by:
   - A significant decrease of the number of microcysts on D28 (-4.4±5.5, p=0.0008);
   - A significant decrease of the number of papules on D28 (-1.5±2.9, p=0.0298);
- Induced an improvement of the skin state in terms of lesions on D28 characterized by:
   - A significant improvement of the lesions number score (-0.8±0.9, p=0.0014),
   - A significant improvement of the lesions visibility score (-0.9±1.3, p=0.0046);
   - A significant improvement of the lesions color intensity score (-0.8±1.6, p=0.0325);
   - A significant improvement of the lesions size score (-0.7±1.2, p=0.0215);
   - A significant improvement of the purifying effect score (-0.9±1.5, p=0.0143);
- Induced an improvement of the skin state in terms of complexion on D28 characterized by:
   - A significant improvement of the stains score (0.8±0.9, p=0.0008),
   - A significant improvement of the redness score (0.7±1.3, p=0.031),
   - A significant improvement of the pores score (1.3±1.5, p=0.0005),
   - A significant improvement of the roughness score (1.1±1.4, p=0.0005),
- Was appreciated by the subjects for its properties and for its efficacy.

Consequently, in comparison to the initial state, the product containing HMA extract presented an anti-acne and purifying efficacy which are confirmed by the decrease of the number of the microcysts and papules and by improvements of the clinical scores of lesions (number, visibility, color intensity, size, purifying effect). The product induced the improvements of the clinical scores of complexion (stains, redness, pores and roughness) thus improved the uniformity of complexion.

After twice daily use for 28 days, a comparison emulsion without 2% HMA extract:
- Did not induce any noticeable change of the number of the acne lesions
- Induced an improvement of the skin state in terms of lesions on D28 characterized by:
   - A significant improvement of the lesions visibility score (-0.9±1.3, p=0.0037);
   - A significant improvement of the lesions color intensity score (-0.9±1.6, p=0.0161),
   - A significant improvement of the lesions size score (-1.4±1.7, p=0.0005),
   - A significant improvement of the purifying effect score (-0.8±1.7, p=0.0202),
- Induced an improvement of the skin state in terms of complexion on D28 characterized by:
   - A significant improvement of the redness score (0.8±1.4, p=0.0177),
   - A significant improvement of the pores score (1±1.4, p=0.0063),
   - A significant improvement of the roughness score (1±2.1, p=0.0427),
- Was appreciated by the subjects for its properties and for its efficacy.

Consequently, in comparison to the initial state, the comparison product presented an anti-acne and purifying efficacy which are confirmed by improvements of the clinical scores of lesions (visibility, color intensity, size, purifying effect). The product induced the improvements of the clinical scores of complexion (redness, pores and roughness) thus improved the uniformity of complexion.

Comparison of the products revealed that the anti-acne efficacy of the product containing HMA extract (in terms of microcysts) was stronger than for the comparison product.

No relevant adverse reaction was observed during the study.

## Claims

1. Composition comprising from 0.005 weight% to 0.250 weight% of *Harungana madagascariensis* leaf extract, based on the weight of the dry extract and of the total weight of the formulation, for its use to treat and/or to prevent acne, in a method of treatment of the human body by therapy.

2. Composition for use according to claim 1, wherein the acne is acne vulgaris and/or juvenile.

3. Composition for use according to claim 1, wherein the extract has a content of polyphenolic compounds as assayed by the Folin-Ciocalteu colorimetric method of ≥ 10 weight % (based on the dry leaf extract).

4. Composition for use according to claim 3, wherein the polyphenolic compounds comprise flavonoids selected from the group of quercitrin, quercetin, glycosylated quercetin, catechin, taxifoline, taxifoline rhamnoside, taxifoline arabinoside, isorhamnetin and/or astilbine.

5. Composition for use according to anyone of claim 1 to 3, wherein said *Harungana madagascariensis* leaf extract comprises less or equal to (≤) 0.03% (weight/volume) of anthraquinone compounds, as determined by HPLC-MS from a 5% solution (weight/volume) of the extract in glycerol and water (60:40 weight/weight).

6. Composition for use according to one of the preceding claims, further comprising one or more topically acceptable excipients and/or one or topically acceptable active ingredients..

7. *Harungana madagascariensis* leaf extract for use to treat and/or to prevent acne, in a method of treatment of the human body by therapy.

## Patentansprüche

1. Zusammensetzung, umfassend von 0,005 Gew.-% bis 0,250 Gew.-% an Extrakt aus *Harungana madagascariensis*-Blättern, basierend auf dem Gewicht des Trockenextrakts und des Gesamtgewichts der Formulierung, zu deren Verwendung bei der Behandlung und/oder Prophylaxe von Akne, in einem Verfahren zur Behandlung des menschlichen Körpers durch Therapie.

2. Zusammensetzung zur Verwendung nach Anspruch 1, wobei es sich bei der Akne um gewöhnliche und/oder um jugendliche Akne handelt.

3. Zusammensetzung zur Verwendung nach Anspruch 1, wobei der Extrakt einen Gehalt an Polyphenolverbindungen, wie nach dem kolorimetrischen Folin-Ciocalteu-Verfahren geprüft, von ≥ 10 Gew.-% (basierend auf dem Trockenblattextrakt) aufweist.

4. Zusammensetzung zur Verwendung nach Anspruch 3, wobei die Polyphenolverbindungen Flavonoide umfassen, die ausgewählt sind aus der Gruppe von Quercitrin, Quercetin, glycosyliertem Quercetin, Catechin, Taxifolin, Taxifolin-Rhamnosid, Taxifolin-Arabinosid, Isorhamnetin und/oder Astilbin.

5. Zusammensetzung zur Verwendung nach einem der Ansprüche 1 bis 3, wobei der Extrakt aus *Harungana madagascariensis*-Blättern weniger oder gleich (≤) 0,03 Gew.-% (Gewicht/Volumen) Anthrachinon-Verbindungen umfasst, wie nach HPLC-MS aus einer 5%igen Lösung (Gewicht/Volumen) des Extrakts in Glycerin und Wasser (60:40 Gewicht/Gewicht) bestimmt.

6. Zusammensetzung zur Verwendung nach einem der vorstehenden Ansprüche, weiter einen oder mehrere topisch verträgliche Hilfsstoffe und/oder einen oder topisch verträgliche Wirkstoffe umfassend.

7. Extrakt aus *Harungana madagascariensis*-Blättern zur Verwendung bei der Behandlung und/oder Prophylaxe von Akne, in einem Verfahren zur Behandlung des menschlichen Körpers durch Therapie.

## Revendications

1. Composition comprenant de 0,005 % en poids à 0,250 % en poids d'extrait de feuilles *d'Harungana madagascariensis,* sur la base du poids de l'extrait sec et du poids total de la formulation, pour son utilisation pour le traitement et/ou la prévention de l'acné, dans un procédé de traitement du corps humain par thérapie.

2. Composition pour son utilisation selon la revendication 1, dans laquelle l'acné est l'acné vulgaire et/ou juvénile.

3. Composition pour son utilisation selon la revendication 1, dans laquelle l'extrait a une teneur en composés polyphénoliques telle que dosée par la méthode colorimétrique de Folin-Ciocalteu ≥ 10 % en poids (sur la base de l'extrait de feuilles sèches).

4. Composition pour son utilisation selon la revendication 3, dans laquelle les composés polyphénoliques comprennent des flavonoïdes sélectionnés dans le groupe consistant en la quercitrine, la quercétine, la quercétine glycosylée, la catéchine, la taxifoline, le rhamnoside de taxifoline, l'arabinoside de taxifoline, l'isorhamnétine et/ou l'astilbine.

5. Composition pour son utilisation selon l'une quelconque des revendications 1 à 3, dans laquelle ledit extrait de feuilles d'*Harungana madagascariensis* comprend 0,03 % (poids/volume) ou moins (≤) de composés d'anthraquinone, tel que déterminé par CLHP-SM d'une solution à 5 % (poids/volume) de l'extrait dans du glycérol et de l'eau (60:40 poids/poids).

6. Composition pour son utilisation selon l'une des revendications précédentes, comprenant en outre un ou plusieurs excipients topiquement acceptables et/ou un ou plusieurs ingrédients actifs topiquement acceptables.

7. Extrait de feuilles d'*Harungana madagascariensis* pour son utilisation dans le traitement et/ou la prévention de l'acné, dans un procédé de traitement du corps humain par thérapie.
